Europäisches Patentamt

European Patent Office

Office européen des brevets

(19)

(11) Publication number: **0 160 015**
**B1**

(12)    — **EUROPEAN PATENT SPECIFICATION**

(45) Date of publication of patent specification: **29.03.89**

(21) Application number: **84903628.0**

(22) Date of filing: **27.09.84**

(86) International application number:
**PCT/DK84/00095**

(87) International publication number:
**WO 85/01441 11.04.85 Gazette 85/09**

(51) Int. Cl.⁴: **A 61 K 35/78,** A 61 K 33/00,
A 61 K 37/54, A 61 K 31/715

(54) **PREPARATION FOR REHYDRATING MONOGASTRIC ANIMALS, INCLUDING HUMAN BEINGS, SUFFERING FROM DIARRHOEA AND USE THEREOF.**

(30) Priority: **03.10.83 DK 4546/83**

(43) Date of publication of application:
**06.11.85 Bulletin 85/45**

(45) Publication of the grant of the patent:
**29.03.89 Bulletin 89/13**

(84) Designated Contracting States:
**AT BE CH DE FR GB LI LU NL SE**

(56) References cited:
**WO-A-82/02650**
**DE-A-2 644 197**
**DE-B-2 455 281**
**DE-B-2 611 979**
**FR-M- 2 996**
**GB-A-1 306 752**
**GB-A-1 509 866**
**US-A-3 449 492**

The file contains technical information
submitted after the application was filed and
not included in this specification

(73) Proprietor: **GJERLOV, Mogens**
**Saksenballe 9 Nr. Broby**
**DK-5672 Broby (DK)**

(72) Inventor: **GJERLOV, Mogens**
**Saksenballe 9 Nr. Broby**
**DK-5672 Broby (DK)**

(74) Representative: **Wiklund, Ingrid Helena et al**
**AWAPATENT AB P.O. Box 5117**
**S-200 71 Malmö (SE)**

(56) References cited:

**US-A-4 083 960**
**US-A-4 164 568**

**Chemical Abstracts, Vol. 98 (1983), abstract
No. 204 438 g, Jpn. Kokai Tokkyo Koho JP 58
39,625 (83 39,625)**

Courier Press, Leamington Spa, England.

# EP 0 160 015 B1

**Description**

The invention relates to a preparation for rehydrating monogastric animals, including human beings, suffering from diarrhoea, especially non-infectious diarrhoea and diarrhoea caused by rota and corona viruses.

Even in well-organized agricultural countries with good veterinary coverage, the mortality among new-born animals such as calves and pigs is still very great. For example, in Denmark in 1980 there were destroyed about 180,000 calves, which corresponds to nearly 20% of the calves born every year. The cause of a number of the deaths among calves, and also of a number of corresponding deaths among sucking pigs, is that they become stressed when they are weaned and placed, for example, in common sties or folds. When the animals are removed and thus no longer receive mother's milk, up to half of the animals develop diarrhoea because they become stressed due to change of fodder, transportation etc. It is assumed that half of the deaths are due to diarrhoea which arises within the first month after their birth. A number of the calves also suffer from rota and corona virus infection, which is a contagious intestinal infection where cows are virus carriers and infect the calves. The disease is caused by ia. strongly reduced production of lactase, so that the animals cannot decompose the lactose in the milk with which they are fed, and hereafter diarrhoea is quickly developed by osmotic effect in the intestinal canal.

New-born calves and pigs with diarrhoea will lose considerable weight because of dehydration and many die.

US Patent Specification No. 4,164,568 deals with a composition for the treatment of diarrhoea (e.g. scours) in domestic animals, e.g. young domestic animals, such as calves.

The composition comprises electrolytes in the form of 7.5—30% of an actively absorbed, naturally occurring amino acid, 0.5—5% of citric acid and 0.1—5% of a non-toxic salt of citric acid as well as 40—80% of an actively absorbed monosaccharide, including glucose. The composition may also comprise other electrolytes, such as sodium chloride, magnesium sulfate, potassium dihydrogen phosphate, calcium gluconate.

The composition is normally administered to the animal after the incidence of diarrhoea in the form of an aqueous solution by the oral route. Such a solution may for example contain 20—45 g/liter of the composition. This Patent Specificaton shows that this composition is useful for treating diarrhoea by rehydration.

The object of the invention is to present a preparation for the treatment of diarrhoea, so that a very large number of the sick animals can be cured in a few days, whereby deaths due to loss of fluid is avoided and for the calves better growth is obtained.

This is achieved by composing the following composition:

A preparation for rehydrating monogastric animals, including human beings, suffering from diarrhoea which preparation is of the kind containing electrolytes and glucose, characterized in that said preparation consists essentially of (a) 20—70% by weight of an absorbent intumescent consisting of Isphagula Husk; (b) 40—60% by weight of electrolytes which electrolytes are a mixture of two or more of the substances magnesium oxide, magnesium carbonate hydroxide, magnesium hydroxide, magnesium silicate, calcium silicate, calcium carbonate, alkali metal chlorides, including sodium and potassium chloride, alkali metal hydrogen carbonates, including sodium and potassium hydrogen carbonate, aluminium phosphate, aluminium hydroxide, citric acid and alkali metal citrates, including sodium and potassium citrate, and in addition to the electrolytes glucose; (c) 0.01—5% by weight a lactase, and in that said preparation optionally consists also of roughage, pectin, starch, one or more taste correctives and/or one or more coloring agents, wherein the amounts of the individual components are chosen in a way so that the sum total of the percentages by weight of the components is 100.00.

Particular embodiments according to the invention are characterized in that at least one of said salts comprised by said electrolytes is at least one of the salts that will replace at least one of the salts lost by diarrhoea,

that roughage is bran of wheat, in that said taste corrective is oil of peppermint, and in that said colouring agent is terra rubrum,

that said electrolytes are composed so as to give a buffer effect in said preparation in solution or in suspension,

that the lactase is a lactase with a pH-optimum between 2 and 10,

that said electrolytes are a mixture of two or more of the substances magnesium hydroxide, sodium chloride, potassium chloride, sodium hydrogen carbonate, citric acid, and in that said lactase is a lactase with a pH-optimum between 5 and 8,

that said preparation consists of 40 percent by weight of Isphagula Husk, 52.925 percent by weight of electrolytes comprising glucose which are made up of 0.575 percent by weight of magnesium oxide, 1.35 percent by weight of citric acid, 2.4 percent by weight of potassium chloride, 2.65 percent by weight of sodium citrate, 4.15 percent by weight of sodium chloride, 6.8 percent by weight of sodium hydrogen carbonate, 35 percent by weight of glucose; 0.025 percent by weight of lactase; 6.8 percent by weight of roughage in the form of bran of wheat; and 0.25 percent by weight of colouring agent in the form of terra rubrum, where the percents by weight are calculated on the basis of the finished preparation,

that said preparation consist of 20—70 percent by weight of Isphagula Husk as absorbent intumescent;

2

40—60 percent by weight of electrolytes which electrolytes are a mixture of two or more of the substances magnesium oxide, magnesium carbonate hydroxide, magnesium silicate, calcium carbonate, alkali metal chlorides, including sodium and potassium chloride, alkali metal hydrogen carbonate, including sodium and potassium hydrogen carbonate, aluminium phosphate, aluminium hydroxide, citric acid and alkali metal citrates, including sodium and potassium citrate, and glucose; and 0.01—5 percent of lactase and the balance is made up of the taste corrective oil of peppermint, the percentages by weight being calculated on the basis of the amounts of the swelling agent, the electrolytes, the glucose, the lactase as well as the balance, and wherein the amounts of the individual components are chosen in a way so that the sum total of the percentages by weight is 100.00,

that said preparation consists of Isphagula Husk being 43 percent by weight of ground, 57 percent by weight of electrolytes which are made up of 0.617 percent by weight magnesium oxide, 1.45 percent by weight of citric acid, 2.575 percent by weight of potassium chloride, 2.845 percent by weight of sodium citrate, 4.456 percent by weight of sodium chloride, 7.3 percent by weight of sodium hydrogen carbonate, 37.58 percent by weight of glucose, 0.027 percent by weight of lactase and 0.2 percent by weight taste corrigent in the form of oil of peppermint where the percentages by weight are calculated on the basis of the finished preparation and where the amounts of the absorbent intumescent Isphagula Husk and the electrolytes are chosen in such a way that the total of the percentages by weight of the individual components is 100.00.

Surprisingly, it has been shown that a mixture of an absorbent intumescent agent, lactose-decomposing enzymes and one or more electrolytes is a quick and effective agent against diarrhoea without any apparent side effects and with a better effect than with the individual components alone. The agent can be used for the treatment of diarrhoea among all offspring of ruminants as long as these are one-stomached, i.e. before they have begun cud-chewing, and for the treatment of non-infectious diarrhoea and diarrhoea caused by rota and corona viruses among all other one-stomached animals, including human beings. The lactose-decomposing enzyme decomposes that lactose which, for example, a calf suffering from diarrhoea is itself unable to decompose and digest. Non-decomposed lactose in the intestinal canal contributes to worsening an attack of diarrhoea. With certain other methods of treatment, it has been suggested that the lactose be decomposed in the milk before the calf receives the milk, but with the preparation according to the invention, the lactose is not decomposed until inside the calf's stomach and intestine which results in fewer side effects, for example in the form of sitiophobia.

The agent according to the invention is produced quite simply by weighing out and mixing the individual parts so that the finished agent is supplied as a dry powder ready for use.

Another great advantage of the preparation according to the invention is that it is possible to cure animals of diarrhoea without the use of normal antibiotics, and thus avoiding the disadvantages herewith in the form of medicinal residues in the animal, and the possibilities of developing bacterial strains resistant to antibiotics.

According to the invention it is very advantageous that the electrolytes in the preparation comprise such salts that replace salt lost by diarrhoea since by rehydration it is merely necessary to administer a preparation which will bring about both rehydration or stop dehydration and provide the lost salts and fluid.

An example of a roughage is bran of wheat, an example of a taste corrective is oil of peppermint and an example of a colouring agent is terra rubrum.

When the preparation comprises a buffer, it is not necessary to protect the enzymes by adopting special measures since the preparation itself for a period of up to 6 hours stabilises the pH-value of the stomach so that the enzymes are not inactivated, and it is not necessary to wait for the dissolution of a coating in the intestine, the enzyme being immediately ready to perform its function. Thus protective measures for the enzyme are also spared.

As stated above it is preferred that the lactase is a lactase having a pH optimum of 2—10.

By composing the preparation wherein the lactase has a pH optimum between 5 and 8 there is obtained a simple preparation which has the required properties, i.e. stopping diarrhoea, providing the necessary salts, providing lactase and bringing it uninfluenced through the stomach in a simple manner.

It is an advantage that the absorbent vegetable fibres swell up in the intestine in a very suitable and natural manner, which gives the contents of the intestine a gelatinous consistency so that the faeces will have a normal consistency already a few hours after the first feeding with the agent. The more quickly that a diarrhoea from which a calf or sucking pig is suffering is brought under control, the greater are the chances of the animal surviving. The glucose content and the necessary salts in the correct amounts will promote the absorption of nutrients and give the weakened animal an easily transformable energy.

Practical applications and experiments have shown that the agent according to the invention is particularly effective when the Isphagula Husk has been ground. If this agent is used immediately when an animal shows the symptoms of diarrhoea, and it is a question of so-called problem stock, then the agent according to the invention should be used in the event of the animal merely refusing to drink, and a case of diarrhoea can normally be stopped merely by treating the animal a few times. It is thus possible to put an immediate stop to the life-threatening loss of water and salts (electrolytes) so that by far the majority of the animals attacked will survive and be restored to health quicker than if they are only treated with electrolytes alone or the water palliative fibres alone. This quicker restoration of the animals will therefore bring about a better growth which has been proved by clinical experiments.

By composing the preparation within the specific weight ratios, as disclosed in claim 7, there is obtained a preparation which will stop diarrhoea among animals and provide rehydration since the preparation will only have to be mixed in water or milk or a water/milk mixture whereupon an animal will willingly drink it.

For human application within the specific weight range the preparation is composed without roughage and dyestuffs but with a corrective having the composition mentioned in claim 8, a composition within specific weight ratios as disclosed in claim 9 being particularly preferred since such a preparation is effective and at the same time has a pleasant taste.

The preparation according to the invention as disclosed is used by pouring 40—55 g of the mixture into lukewarm water, milk or a water/milk mixture at around 38°C. The result is a mixture which the animals are very willing to drink and which quickly cures them of diarrhoea because the gel formed by the agent in the intestinal canal has the following characteristics:

a) a protective effect on the actual intestinal mucosa,

b) binds some of the bacteria and their toxins to itself,

c) ensures a normal intestinal passage (peristalsis),

d) quickly stops the loss of fluid and electrolytes,

e) buffers the capacity due to the added electrolytes in the gel formed, protects the added lactase from inactivation for up to 6 hours, and

f) the lactase decomposes the lactose in the intestine and thus recreates a normal osmotic balance.

For human application the same results as mentioned above will be obtained. If desired, cold liquid can be used for the preparation.

Practical experiment with the preparation according to the invention.

480 sucking calves, all of them two to three weeks old, were taken in for rearing experiments under uniform optimum conditions with regard to hygiene, climate and feeding, the object being to test the effect of the preparation on dietetic-conditioned diarrhoea among sucking calves.

By the first feeding after the calves taken in had been placed in the cow-house at the research station, all of the calves each received three litres of a conventional electrolyte/water mixture, after which this was gradually changed during the course of five days to up to 7 litres of milk substitute. The calves also had free access to hay and ordinary fodder supplements.

During the course of fourteen days from the time they were taken in, 86 of the calves, i.e. 17.9%, contracted stomach/intestinal disturbances. These animals were immediately treated with the preparation according to the invention and as disclosed in claim 12.

73 of the sick calves, i.e. 84.9%, were completely cured within a few days. The 13 calves which were not cured immediately by the treatment were then given supplementary treatment with antibiotics.

From this it will be seen that the diarrhoea which is contracted early by many calves fattened on full milk and by sucking pigs is often due to virus infections and to transport stress, stress as a result of feeding change and stress from changed environment, and can therefore be cured with the preparation according to the invention without the use of antibiotics. Only 13 animals out of 86, i.e. approx. 15%, required supplementary antibiotic treatment.

Course of treatment when using the preparation.

The agent is mixed in water and is dosed in accordance with the weight of the animal, the amounts used being as stated in the following table:

| Weight of animal | Dose per feeding | No. of feeds per 24 hours |
|---|---|---|
| approx. 20 kg | $\frac{1}{2}$ l water + approx. 25 g | 4 |
| approx. 30 kg | 1 l water + approx. 50 g | 4 |
| approx. 40 kg | $1\frac{1}{2}$ l water + approx. 75 g | 3—4 |
| approx. 50 kg | 2 l water + approx. 100 g | 3 |
| approx. 60 kg | $2\frac{1}{2}$ l water + approx. 125 g | 3 |

After the first twenty-four hours, it can be an advantage to add 25—50 g curdled milk product, for example soured milk, yoghurt or junket per litre. From the third day, one can gradually change over to the normal mixed fodder.

There are also cattle stock among which stomach/intestine disturbances are a recurrent problem. In such cases it can be an advantage to give the calves the preparation according to the invention as soon as they refuse to drink the normal feed, for example consisting of full-cream milk or other milk mixtures.

4

Example of Comparison

The curative properties against diarrhoea of the preparation according to the invention were compared by a controlled experiment with the properties of Calmix neo® at a Dutch calf fattening station.

For the experiments there were used in all 230 calves which were divided in 5 groups of 46 animals each. They were placed in wooden pens with floor grating and ventilation. One week old calves were used. The animals were weighed before the experiment, after 29 days and after 58 days. At the beginning there is given 1.5 l water and 75 g electrolytes as first feeding. The fodder consisted of a bag of Heftica® per calf followed by Hemeka® start and fattening according to the usual schedule.

In case of diarrhoea either the preparation according to the invention or Calmix neo® is given.

The experiment results are given in the table below.

| Average results Curative treatment | Conservative treatment (Calmix neo®) | Preparation according to the invention |
|---|---|---|
| No. of calves | 15 | 15 |
| Average weight at start | 41.6 kg | 38.3 kg |
| after 29 days | 47.5 kg | 45.3 kg |
| 58 days | 74.4 kg | 73.7 kg |
| Average weight gain after 29 days | 5.9 kg | 7.0 kg |
| 58 days | 32.8 kg | 35.4 kg |
| Average weight gain after 29 days | 5.9 kg | 7.0 kg |
| 29—58 days | 26.9 kg | 28.4 kg |
| Average weight gain/day after 29 days | 203 g | 241 g |
| 59 days | 565 g | 610 g |

The curative properties of the preparation prove to be good. After 58 days the calves showed an average of 2.6 kg larger growth than the control group which was treated with Calmix neo®. Moreover, the average weight per day among the animals which were treated with the preparation according to the invention, increased in the second period more than the animals treated with Calmix neo®. This shows that not only are the absorption and the digestive capacity of the intestine less influenced but they are at the same time more quickly restored. This result supports the fact that the pharmacokinetics of the preparation according to the invention builds on a protection of the intestinal wall against pathogenic attacks coupled with a compensation for lack of lactase by means of acid resistant lactase whereby the secretion as well as the osmotic component are combatted.

# EP 0 160 015 B1

## Example 1

The preparation according to the invention can be composed, for example, as follows:

1000 g contains:

| | | |
|---|---|---|
| Magnesium oxide | 5.75 g | |
| Citric acid | 13.50 g | |
| Postassium chloride | 24.00 g | |
| Sodium citrate | 26.50 g | electrolytes |
| Sodium chloride | 41.50 g | |
| Sodium bicarbonate | 68.00 g | |
| Glucose | 350.00 g | |
| Terra rubrum | 2.50 g | dyestuff |
| Wheat bran | 68.00 g | roughage |
| Isphagula Husk (dried seed coats of plantago ovata) | 400.00 g | absorbent fibre |
| Lactase | 0.25 g | enzyme |
| Total | 1000.00 g | |

The individual ingredients, all of which are available as dry powders, are mixed mechanically and are thereafter immediately ready for use.

The agent according to the invention must not be administered in dry form, but must be suspended in water and administered as a solution or suspension. The intumescence occurs hereafter in the intestinal canal during a suitable period, whereby by absorption of fluid said intumescent swells up and gives the contents of the intestine a suitable consistency, and binds and receives some of the bacteria and their toxins so that a diarrhoea is at once stopped. An intumescent being capable of being swollen up by water may be described as a water binding swelling agent.

## Example 2

The preparation according to the invention can also have the following composition which is particularly preferred for human application:

1000 g contains:

| | |
|---|---|
| Magnesium oxide | 6.17 g |
| Citric acid | 14.50 g |
| Potassium chloride | 25.75 g |
| Sodium citrate | 28.45 g |
| Sodium chloride | 44.56 g |
| Sodium bicarbonate | 73.00 g |
| Glucose | 375.80 g |
| Isphagula Husk, crushed | 429.50 g |
| Lactase | 0.27 g |
| Oil of peppermint | 2.00 g |

## Example 3

A suspension or solution of the preparation prepared in example 1 is produced by mixing 50 g preparation to 1 litre of water. The pH-value in the fresh preparation is 8.56. By filtration to a pH-value of 5.73 there is used 40.5 meq hydrochloric acid which shows that there is not inconsiderable Buffer effect in the preparation which will "neutralise" the hydrochloric acid in the stomach and consequently protect the lactase.

6

The chemicals used in the examples are ordinary commercial chemicals and the lactase is preferably CHBS lactase 25.000 from Chr. Hansens Laboratorium, Copenhagen.

**Claims**

1. A preparation for rehydrating monogastric animals, including human beings, suffering from diarrhoea which preparation is of the kind containing electrolytes and glucose, characterized in that said preparation consists essentially of (a) 20—70% by weight of an absorbent intumescent consisting of Isphagula Husk; (b) 40—60% by weight of electrolytes which electrolytes are a mixture of two or more of the substances magnesium oxide, magnesium carbonate hydroxide, magnesium hydroxide, magnesium silicate, calcium silicate, calcium carbonate, alkali metal chlorides, including sodium and potassium chloride, alkali metal hydrogen carbonates, including sodium and potassium hydrogen carbonate, aluminium phosphate, aluminium hydroxide, citric acid and alkali metal citrates, including sodium and potassium citrate, and in addition to the electrolytes glucose; (c) 0.01—5% by weight a lactase, and in that said preparation optionally consists also of roughage, pectin, starch, one or more taste correctives and/or one or more colouring agents, wherein the amounts of the individual components are chosen in a way so that the sum total of the percentages by weight of the components is 100.00.

2. The preparation according to claim 1, characterized in that at least one of said salts comprised by said electrolytes is at least one of the salts that will replace at least one of the salt lost by diarrhoea.

3. The preparation according to claim 1, characterized in that roughage is bran of wheat, in that said taste corrective is oil of peppermint, and in that said colouring agent is terra rubrum.

4. The preparation according to claim 1, characterized in that said electrolytes are composed so as to give a buffer effect in said preparation in solution or in suspension.

5. The preparation according to claim 1, characterized in that the lactase is a lactase with a pH-optimum between 2 and 10.

6. The preparation according to claim 5, characterized in that said electrolytes are a mixture of two or more of the substances magnesium hydroxide, sodium chloride, potassium chloride, sodium hydrogen carbonate, citric acid, and in that said lactase is a lactase with a pH-optimum between 5 and 8.

7. The preparation according to any of the preceding claims, characterized in that said preparation consists of 40 percent by weight of Isphagula Husk, 52.925 percent by weight of electrolytes comprising glucose which are made up of 0.575 percent by weight of magnesium oxide, 1.35 percent by weight of citric acid, 2.4 percent by weight of potassium chloride, 2.65 percent by weight of sodium citrate, 4.15 percent by weight of sodium chloride, 6.8 percent by weight of sodium hydrogen carbonate, 35 percent by weight of glucose; 0.025 percent by weight of lactase; 6.8 percent by weight of roughage in the form of bran of wheat; and 0.25 percent by weight of colouring agent in the form of terra rubrum, where the percent by weight are calculated on the basis of the finished preparation.

8. Preparation according to any of the claims 1 to 6, characterized in that said preparation consist of 20—70 percent by weight of Isphagula Husk as absorbent intumescent; 40—60 percent by weight of electrolytes which electrolytes are a mixture of two or more of the substances magnesium oxide, magnesium carbonate hydroxide, magnesium silicate, calcium carbonate, alkali metal chlorides, including sodium and potassium chloride, alkali metal hydrogen carbonate, including sodium and potassium hydrogen carbonate, aluminium phosphate, aluminium hydroxide, citric acid and alkali metal citrates, including sodium and potassium citrate, and glucose; and 0.01—5 percent of lactase and the balance is made up of the taste corrective oil of peppermint, the percentages by weight being calculated on the basis of the amounts of the swelling agent, the electrolytes, the glucose, the lactase as well as the balance, and wherein the amounts of the individual components are chosen in a way so that the sum total of the percentages by weight is 100.00.

9. The preparation according to claim 8, characterized in that said preparation consists of 43 percent by weight of Isphagula Husk being ground, 57 percent by weight of electrolytes which are made up of 0.617 percent by weight magnesium oxide, 1.45 percent by weight of citric acid, 2.575 percent by weight of potassium chloride, 2.845 percent by weight of sodium citrate, 4.456 percent by weight of sodium chloride, 7.3 percent by weight of sodium hydrogen carbonate, 37.58 percent by weight of glucose, 0.027 percent by weight of lactase and 0.2 percent by weight taste corrective in the form of oil of peppermint where the percentages by weight are calculated on the basis of the finished preparation and where the amounts of the absorbent intumescent Isphagula Husk and the electrolytes are chosen in such a way that the total of the percentages by weight of the invididual components is 100.00.

**Patentansprüche**

1. Zubereitung zum Rehydrieren von monogastrischen Tieren einschließlich des Menschen, die an Diarrhoe leiden, wobei die Zubereitung Elektrolyte und Glucose enthält, dadurch gekennzeichnet, daß die Zubereitung im wesentlichen umfaßt:

(a) 20 bis 70 Gew.-% eines anschwellenden Absorptionsmittels, bestehend aus Isphagula Husk;

(b) 40 bis 60 Gew.-% Elektrolyte, wobei die Elektrolyte eine Mischung sind aus zwei oder mehreren der Substanzen Magensiumoxid, Magnesiumcarbonathydroxid, Magnesiumhydroxid, Magnesiumsilicat, Cal-

EP 0 160 015 B1

ciumsilicat, Calciumcarbonat, Alkalimetallchloriden, einschließlich Natrium- und Kaliumchlorid, Alkalimetallhydrogencarbonaten, einschließlich Natriumund Kaliumhydrogencarbonat, Aluminium- phosphat, Aluminiumhydroxid, Zitronensäure und Alkalimetallcitraten, einschließlich Natrium- und Kaliumcitrat, und zusätzlich zu den Elektrolyten Glucose;

(c) 0,01 bis 5 Gew.-% Lactase;

weiterhin dadurch gekennzeichnet, daß die Zubereitung wahlweise außerdem Rauhfutter, Pektin, Stärke, ein oder mehrere Geschmackskorrektivmittel und/oder ein oder mehrere Färbemittel umfaßt, wobei die Mengen der einzelnen Bestandteile so gewählt sind, daß die Gesamtsumme der Gewichtsprozente der Bestandteile 100 ist.

2. Zubereitung nach Anspruch 1, dadurch gekennzeichnet, daß mindestens eines der Salze, die von den genannten Elektrolyten umfaßt werden, mindestens eines der Salze ist, das mindestens eines der durch die Diarrhoe verlorenen Salze ersetzen wird.

3. Zubereitung nach Anspruch 1, dadurch gekennzeichnet, daß das Rauhfutter Weizenkleie, das Geschmackskorrektivmittel Pfefferminzöl und das Färbemittel Terr rubrum ist.

4. Zubereitung nach Anspruch 1, dadurch gekennzeichnet, daß die Elektrolyte so zusammengesetzt sind, daß sie in Lösung oder in Suspension eine Pufferwirkung in der Zubereitung haben.

5. Zubereitung nach Anspruch 1, dadurch gekennzeichnet, daß die Lactase eine Lactase mit einem pH-Optimum zwischen 2 und 10 ist.

6. Zubereitung nach Anspruch 5, dadurch gekennzeichnet, daß die Elektrolyte eine Mischung aus zwei oder mehreren der Substanzen Magnesiumhydroxid, Natriumchlorid, Kaliumchlorid, Natriumhydrogencarbonat und Zitronensäure sind, und daß die Lactase eine Lactase mit einem pH-Optimum zwischen 5 und 8 ist.

7. Zubereitung nach mindestens einem der vorstehenden Ansprüche, dadurch gekennzeichnet, daß die Zubereitung aus 40 Gew.-% Isphagula Husk, 52,925 Gew.-% Glucose und Elektrolyten, die aus 0,575 Gew.-% Magnesiumoxid, 1,35 Gew.-% Zitronensäure, 2,4 Gew.-% Kaliumchlorid, 2,65 Gew.-% Natriumcitrat, 4,15 Gew.-% Natriumchlorid, 6,8 Gew.-% Natriumhydrogencarbonat und 35 Gew.-% Glucose zusammengesetzt sind, 0,025 Gew.-% Lactase, 6,8 Gew.-% Rauhfutter in Form von Weizenkleie und 0,25 Gew.-% Färbemittel in Form von Terra rubrum besteht, wobei die Gewichtsprozente auf der Grundlage der fertiggestellten Zubereitung berechnet sind.

8. Zubereitung nach mindestens einem der Ansprüche 1 bis 6, dadurch gekennzeichnet, daß die Zubereitung 20 bis 70 Gew.-% Isphagula Husk als anschwellendes Absorptionsmittel; 40 bis 60 Gew.-% Elektrokyte, wobei die Elektrolyte eine Mischung aus 2 oder mehreren der Substanzen Magnesiumoxid, Magnesiumcarbonathydroxid, Magnesiumsilicat, Calciumcarbonat, Alkalimetallchloride einschließlich Natrium- und Kaliumchlorid, Alkalimetallhydrogencarbonäten, einschließlish Natrium- und Kaliumhydrogencarbonat, Aluminiumphosphat, Aluminiumhydroxid, Zitronensäure und Alkalimetallcitraten, einschließlich Natrium- und Kaliumcitrat, und Glucose sind; und 0,01 bis 5 Gew.-% Lactase enthält und die Restmenge aus dem Geschmackskorrektivmittel Pfefferminzöl besteht; die Gewichtsprozente sind auf der Grundlage der Mengen des anschwellenden Mittels, der Elektrolyte, der Glucose, der Lactase sowie der Restmenge berechnet, wobei die Mengen der einzelnen Bestandteils so gewählt sind, daß die Gesamtsumme der Gewichtsprozente 100 beträgt.

9. Zubereitung nach Anspruch 8, dadurch gekennzeichnet, daß die Zubereitung aus 43 Gew.-% gemahlenem Isphagula Husk, 57 Gew.-% Elektrolyten, wobei die Elektrolyte aus 0,617 Gew.-% Magnesiumoxid, 1,45 Gew.-% Zitronensäure, 2,575 Gew.-% Kaliumchlorid, 2,845 Gew.-% Natriumcitrat, 4,456 Gew.-% Natriumchlorid, 7,3 Gew.-% Natriumhydrogencarbonat, 37,58 Gew.-% Glucose zusammengesetzt sind, 0,027 Gew.-% Lactase und 0,2 Gew.-% Geschmackskorrektivmittel in Form von Pfefferminzöl besteht, wobei die Gewichtsprozente auf der Grundlage der fertiggestellten Zubereitung berechnet und die Mengen des anschwellenden Absorptionsmittels Isphagula Husk und der Elektrolyte so gewählt sind, daß die Gesamtsumme der Gewichtsprozente der einzelnen Bestandteile 100 beträgt.

**Revendications**

1. Une préparation destinée à la réhydratation des animaux à estomac unique, dits monogastriques, y compris l'homme, souffrant de diarrhée, ladite préparation étant u type contenant des électrolytes et du glucose et se caractérisant par le fait qu'elle est composée essentiellement de:

(a) 20 à 70% en poids d'un intumescent absorbant constitué d'Isphagula Husk;

(b) 40 à 60% en poids d'électrolytes, lesdits électrolytes consistant en un mélange de deux ou plusieurs des substances suivantes: oxyde de magnésium, hydrogénocarbonate de magnésium, hydroxyde de magnésium, silicate de magnésium, silicate de calcium, carbonate de calcium, chlorures de métaux alcalins, y compris chlorures de sodium et de potassium, hydrogénocarbonates de métaux alcalins, y compris hydrogénocarbonates de sodium et de potassium, phosphate d'aluminium, hydroxyde d'aluminium, acide citrique et citrates de métaux alcalins, y compris citrates de sodium et de potassium et, en plus des électrolytes, du glucose;

(c) 0,01 à 5% en poids de lactase, ladite préparation pouvant éventuellement contenir également un ballast intestinal, de la pectine, de l'amidon, un ou plusieurs correcteurs de goût et/ou un ou plusieurs colorants, les quantités des ingrédients individuels étant déterminées de manière que le total des

8

pourcentages en poids desdits ingrédients soit égal à 100,00.

2. La préparation selon la revendication 1, se caractérisant par le fait qu'au moins un desdits sels compris dans lesdits électrolytes est au moins un des sels qui remplacera au moins un des sels perdus par la diarrhée.

3. La préparation selon la revendication 1, se caractérisant par le fait que le ballast intestinal est du son de blé, que ledit correcteur de goût est de l'essence de menthe poivrée et que ledit colorant est de la terre rubrum.

4. La préparation selon la revendication 1, se caractérisant par le fait que lesdits électrolytes sont composés de manière à conférer un effet tampon à ladite préparation en solution ou en suspension.

5. La préparation selon la revendication 1, se caractérisant par le fait que la lactase est une lactase à pH optimum compris entre 2 et 10.

6. La préparation selon la revendication 5, se caractérisant par le fait que lesdits électrolytes sont un mélange de deux ou plusieurs des substances suivantes: hydroxyde de magnésium, chlorure de sodium, chlorure de potassium, hydrogénocarbonate de sodium, acide citrique et dans le fait que ladite lactase est une lactase à pH optimum compris entre 5 et 8.

7. La préparation selon l'une quelconque des revendications qui précèdent, se caractérisant par le fait que ladite préparation est constituée de 40 pour cent en poids d'Isphagula Husk, 52,925 pour cent en poids d'électrolytes renfermant du glucose, qui sont constitués de 0,575 pour cent en poids d'oxyde de magnésium, 1,35 pour cent en poids d'acide citrique, 2,4 pour cent en poids de chlorure de potassium, 2,65 pour cent en poids de citrate de sodium, 4,15 pour cent en poids de chlorure de sodium, 6,8 pour cent en poids d'hydrogénocarbonate de sodium, 35 pour cent en poids de glucose, 0,025 pour cent en poids de lactase; 6,8 pour cent en poids de ballast intestinal sous la forme de son de blé; et de 0,25 pour cent en poids de colorant sous la forme de terre rubrum, les pourcentages en poids étant calculés sur la base de la préparation finie.

8. La préparation selon l'une quelconque des revendications 1 à 6, se caractérisant par le fait que ladite préparation est composée de 20 à 70 pour cent en poids d'Isphagula Husk en tant qu'intumescent absorbant; de 40 à 60 pour cent en poids d'électrolytes, lesdits électrolytes étant un mélange de deux ou plusieurs des substances suivantes: oxyde de magnésium, hydrogénocarbonate de magnésium, silicate de magnésium, carbonate de calcium, chlorures de métaux alcalins, y compris chlorures de sodium et de potassium, hydrogénocarbonates de métaux alcalins, y compris hydrogénoca'rbonates de sodium et de potassium, phosphate d'aluminium, hydroxyde d'aluminium, acide citrique et citrates de métaux alcalins, y compris citrates de sodium et de potassium et glucose; et de 0,01 à 5 pour cent de lactase, la balance étant constituée du correcteur de goût, l'essence de menthe poivrée et les pourcentages en poids étant calculés sur la base des quantités d'agent gonflant, d'électrolytes, de glucose, de lactase ainsi que de balance, et dans laquelle les quantités des différents ingrédients sont déterminées de manière que le total des pourcentages en poids soit égal à 100,00.

9. La préparation selon la revendication 8, se caractérisant par le fait que ladite préparation est composée de 43 pour cent en poids d'Isphagula Husk broyé et de 57 pour cent en poids d'électrolytes, constitués de 0,617 pour cent en poids d'oxyde de magnésium, de 1,45 pour cent en poids d'acide citrique, de 2,575 pour cent en poids de chlorure de potassium, de 2,845 pour cent en poids de citrate de sodium, de 4,456 pour cent en poids de chlorure de sodium, de 7,3 pour cent en poids d'hydrogénocarbonate de sodium, de 37,58 pour cent en poids de glucose, de 0,027 pour cent en poids de lactase et de 0,2 pour cent en poids de correcteur de goût sous la forme d'essence de menthe poivrée, les pourcentages en poids étant calculés sur la base de la préparation finie et les quantites d'intumescent absorbant, Isphagula Husk et d'électrolytes étant déterminées de manière que le total des pourcentages en poids des différents ingrédients soit égal à 100,00.